(19) Europäisches Patentamt
European Patent Office
Office européen des brevets

(11) EP 1 995 244 A1

(12) **EUROPEAN PATENT APPLICATION**
published in accordance with Art. 158(3) EPC

(43) Date of publication:
**26.11.2008 Bulletin 2008/48**

(21) Application number: **07738759.5**

(22) Date of filing: **09.03.2007**

(51) Int Cl.:
***C07D 303/30*** *(2006.01)* ***C07D 301/32*** *(2006.01)*

(86) International application number:
**PCT/JP2007/055312**

(87) International publication number:
**WO 2007/105808 (20.09.2007 Gazette 2007/38)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **15.03.2006 JP 2006070392**

(71) Applicant: **Sumitomo Chemical Company, Limited**
**Tokyo 104-8260 (JP)**

(72) Inventor: **TANAKA, Shinya**
**Toyono-gun**
**Osaka 563-0103 (JP)**

(74) Representative: **Vossius & Partner**
**P.O. Box 86 07 67**
**81634 München (DE)**

(54) **METHOD FOR CRYSTALLIZATION OF EPOXY COMPOUND**

(57) A process for crystallizing an epoxy compound, which comprises the steps of:
(A) cooling a solution containing a crude product of an epoxy compound, which contains as a main component the epoxy compound represented by the formula (1):

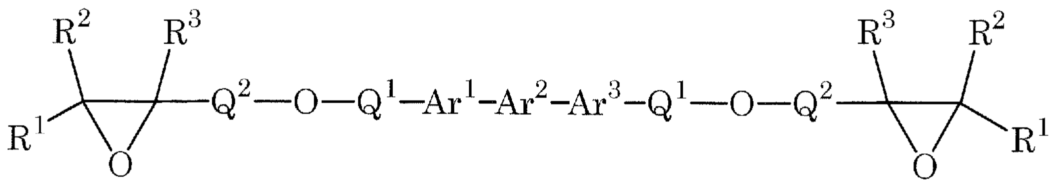

(1)

wherein $Ar^1$, $Ar^2$ and $Ar^3$ are the same or different and each denotes any one of divalent groups represented by the following formulas:

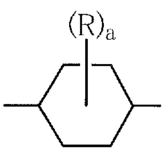
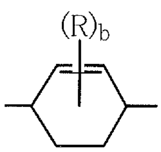
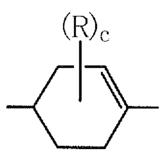
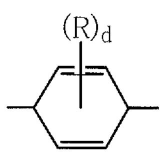

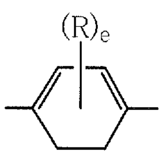
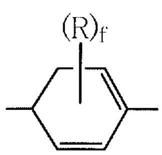
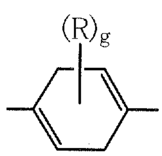
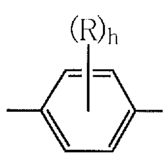

to an internal temperature $T^1$°C to precipitate crystals of the epoxy compound represented by the formula (1), thus obtaining a suspension;

EP 1 995 244 A1

(B) heating the suspension obtained in the step (A) to a temperature $T^2$°C which satisfies the formula (I):

$$T^1 < T^2 < T \quad (I)$$

wherein T denotes a saturation temperature of the epoxy compound in the solution containing a crude product of the epoxy compound used in the step (A); and
(C) cooling the suspension heated to the temperature $T^2$°C in the step (B) to a temperature $T^3$°C which satisfies the following formula (II).

$$T^3 < T^1 \quad (II)$$

## Description

### Technical Field

The present invention relates to a process for crystallizing an epoxy compound.

### Background Art

An epoxy compound represented by the formula (1):

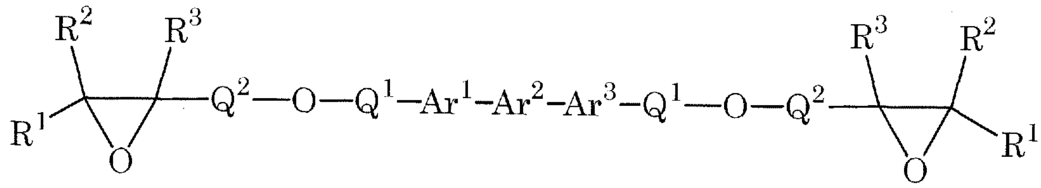

(1)

wherein,

$Q^1$ denotes a single bond or a straight-chain alkylene group having 1 to 9 carbon atoms, in which at least one methylene group composing the straight-chain alkylene group is optionally substituted with an alkyl group having 1 to 8 carbon atoms and -O- or -N ($R^4$)- is optionally inserted between the methylene groups, in which $R^4$ denotes hydrogen atom or an alkyl group having 1 to 8 carbon atoms;
$Q^2$ denotes a straight-chain alkylene group having 1 to 8 carbon atoms, in which at least one methylene group composing the straight-chain alkylene group is optionally substituted with an alkyl group having 1 to 8 carbon atoms;
$Ar^1$, $Ar^2$ and $Ar^3$ are the same or different and each denotes any one of divalent groups represented by the following formulas:

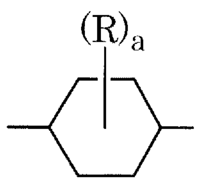
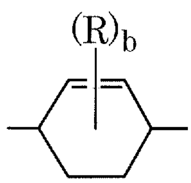
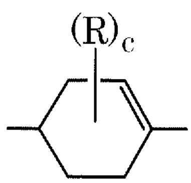
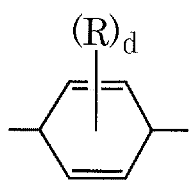

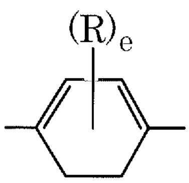
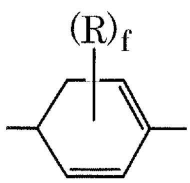
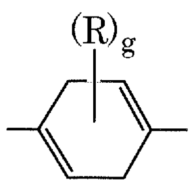
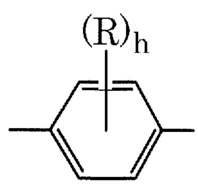

in which R denotes an alkyl group having 1 to 8 carbon atoms, a denotes an integer of 0 to 8, b, e and g denotes an integer of 0 to 6, c denotes an integer of 0 to 7, d and h denote an integer of 0 to 4, and f denotes an integer of 0 to 5, and when more than one R exists in said divalent group, all of R may be the same group or different groups; and

$R^1$, $R^2$ and $R^3$ are the same or different and each denotes hydrogen atom or an alkyl group having 1 to 8 carbon atoms; had a lower melt temperature and can be melt-blended with a curing agent at a curing temperature or lower, as described in EP 1698625 A1. A cured epoxy resin obtained by curing the epoxy compound with the use of a curing agent not only exhibits liquid crystallinity, but also has a high thermal conductivity, so that it is useful as an insulating material which requires a high heat dissipation capacity, such as a printed wiring board.

EP 1698625 A1 discloses a method comprising removing an unreacted epihalohydrin from a reaction mixture

containing an epoxy compound, adding a solvent to obtain a solution, and cooling the solution to precipitate crystals of the epoxy compound.

However, a further industrial improvement has been required from the point of view of purity and filterability of the obtained epoxy compound.

Disclosure of Invention

The present invention provides:

<1> a process for crystallizing an epoxy compound, which comprises the steps of:

(A) cooling a solution containing a crude product of an epoxy compound, which contains as a main component an epoxy compound represented by the formula (1):

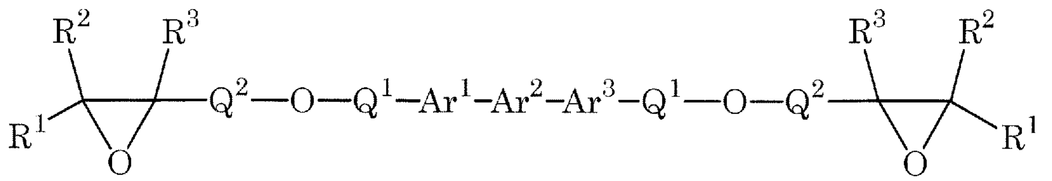

(1)

wherein

$Ar^1$, $Ar^2$ and $Ar^3$ are the same or different and each denotes any one of divalent groups represented by the following formulas:

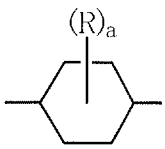 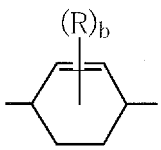 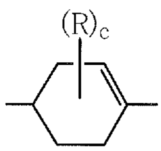 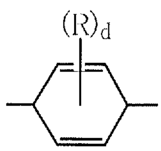

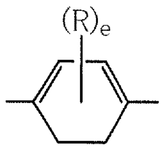 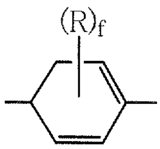 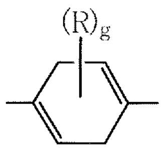 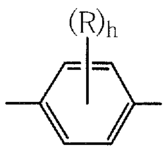

in which R denotes an alkyl group having 1 to 8 carbon atoms, a denotes an integer of 0 to 8, b, e and g denote an integer of 0 to 6, c denotes an integer of 0 to 7, d and h denote an integer of 0 to 4, and f denotes an integer of 0 to 5, and when more than one R exists in said divalent group, all of R may be the same group or different groups;

$R^1$, $R^2$ and $R^3$ are the same or different and each denotes hydrogen atom or an alkyl group having 1 to 8 carbon atoms;

$Q^1$ denotes a single bond or a straight-chain alkylene group having 1 to 9 carbon atoms, in which at least one methylene group composing the straight-chain alkylene group is optionally substituted with an alkyl group having 1 to 8 carbon atoms and -O- or -N($R^4$)- is optionally inserted between the methylene groups, in which $R^4$ denotes hydrogen atom or an alkyl group having 1 to 8 carbon atoms; and

$Q^2$ denotes a straight-chain alkylene group having 1 to 8 carbon atoms, in which at least one methylene group composing the straight-chain alkylene group is optionally substituted with an alkyl group having 1 to 8 carbon atoms, to an internal temperature $T^1$°C to precipitate crystals of the epoxy compound represented by the formula (1), thus obtaining a suspension;

(B) heating the suspension obtained in the above-described step (A) ;
to a temperature $T^2$°C which satisfies the formula (I):

$$T^1 < T^2 < T \qquad (I)$$

wherein T denotes a saturation temperature of the epoxy compound in the solution containing a crude product of the epoxy compound used in the above-described step (A); and
(C) cooling the suspension heated to the temperature $T^2$°C in the above-described step (B) to a temperature $T^3$°C which satisfies the following formula (II):

$$T^3 < T^1 \qquad (II);$$

<2> the process for crystallizing an epoxy compound according to <1>, wherein a solvent of the solution containing a crude product of the epoxy compound is at least one selected from the group consisting of an aliphatic ketone solvent, an ether solvent and an aromatic hydrocarbon solvent;
<3> the process for crystallizing an epoxy compound according to <1> or <2>, wherein the temperature T°C is from 30 to 60°C; <4> the process for crystallizing an epoxy compound any one of <1> to <3>, wherein the concentration of the epoxy compound in the solution containing a crude product of the epoxy compound is from 10 to 40% by weight and the temperature $T^1$ is a temperature which satisfies the following formula (III):

$$T^1 < T - 5 \qquad (III);$$

<5> The process for crystallizing an epoxy compound according to any one of <1> to <4>, wherein the temperature $T^2$ is a temperature which satisfies the following formula (IV):

$$T - 5 < T^2 < T \qquad (IV);$$

<6> the process for crystallizing an epoxy compound according to any one of <1> to <5>, wherein the temperature $T^3$ is a temperature which satisfies the following formula (V):

$$-10 < T^3 < T - 5 \qquad (V);$$

<7> the process for crystallizing an epoxy compound according to any one of <1> to <6>, wherein the temperature $T^1$ is from 10 to 30°C;
<8> the process for crystallizing an epoxy compound according to any one of <1> to <7>, wherein the temperature $T^2$ is from 30 to 60°C;
<9> the process for crystallizing an epoxy compound according to any one of <1> to <8>, wherein the temperature $T^3$ is from -10 to 20°C;
<10> the process for crystallizing an epoxy compound according to any one of <1> to <9>, wherein $Ar^1$ and $Ar^3$ independently denote a group represented by the following formula:

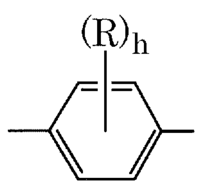

wherein R and h are as defined above;
<11> the process for crystallizing an epoxy compound according to any one of <1> to <10>, wherein $Ar^1$ and $Ar^3$ independently denote 1,4-phenylene group, 3-methyl-1,4-phenylene group or 3-isopropyl-1,4-phenylene group;

<12> the process for crystallizing an epoxy compound according to any one of <1> to <11>, wherein $Ar^2$ is a group represented by the following formula:

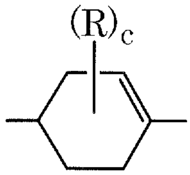

wherein R and c are as defined above;

<13> the process for crystallizing an epoxy compound according to any one of <1> to <12>, wherein $Ar^2$ is 1-cyclohexene-1,4-diyl group;

<14> the process for crystallizing an epoxy compound according to any one of <1> to <9>, wherein $Ar^1$ and $Ar^3$ independently denote a group represented by the following formula:

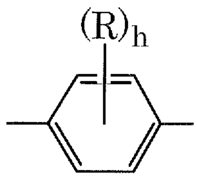

wherein R and h are as defined above, and $Ar^2$ denotes a group represented by the following formula:

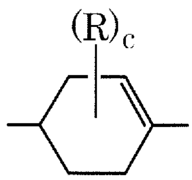

wherein R and c are as defined above; and

<15> the process for crystallizing an epoxy compound according to any one of <1> to <9>, wherein $Ar^1$ and $Ar^3$ independently denote 1,4-phenylene group, 3-methyl-1,4-phenylene group or 3-isopropyl-1,4-phenylene group, and $Ar^2$ denotes 1-cyclohexene-1,4-diyl group.

Best Mode for Carrying Out the Invention

First, the step (A) will be described.

In the formula of the epoxy compound represented by the formula (1):

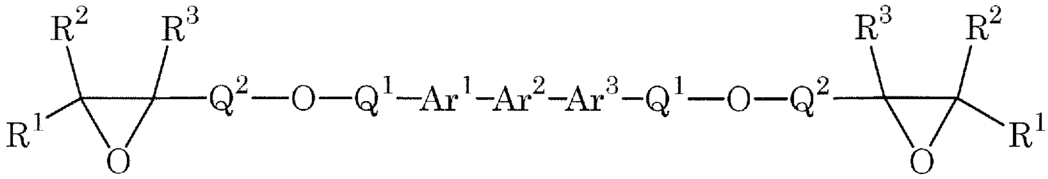

(1)

(hereinafter abbreviated to the epoxy compound (1)),

$Ar^1$, $Ar^2$ and $Ar^3$ are the same or different and each denotes any one of divalent groups represented by the following formulas:

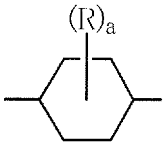 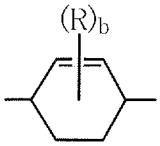 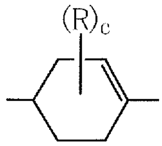 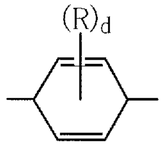

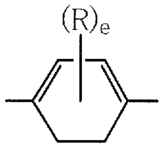 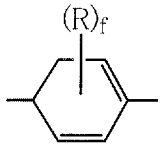 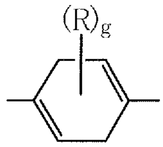 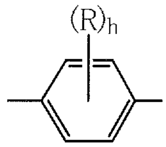

in which R denotes alkyl group having 1 to 8 carbon atoms, a denotes an integer of 0 to 8, b, e and g denote an integer of 0 to 6, c denotes an integer of 0 to 7, d and h denote an integer of 0 to 4, and f denotes an integer of 0 to 5.

Examples of the alkyl group having 1 to 8 carbon atoms include straight-chain, branched chain or cyclic alkyl groups such as methyl, ethyl, n-propyl, isopropyl, n-butyl, isobutyl, sec-butyl, tert-butyl, n-pentyl, tert-pentyl, n-hexyl, 1-methylpentyl, 1-ethylbutyl, n-heptyl, n-octyl, 2-ethylhexyl, isooctyl, tert-octyl, cyclohexyl and cyclooctyl group.

When more than one R exists in said divalent group, all of R may be the same group or different groups.

Examples of the divalent group include cyclohexane-1,4-diyl, 2-cyclohexene-1,4-diyl, 1-cyclohexene-1,4-diyl, 1,4-cyclohexadiene-3,6-diyl, 1,3-cyclohexadiene-1,4-diyl, 1,3-cyclohexanediene-2,5-diyl, 1,4-cyclohexanediene-1,4-diyl, 1,4-phenylene, 2-methylcyclohexane-1,4-diyl, 3-methyl-1,4-phenylene and 3-isopropyl-1,4-phenylene group.

Among the epoxy compounds (1), preferred is an epoxy compound (1) in which $Ar^1$ and $Ar^3$ independently denote a group represented by the following formula:

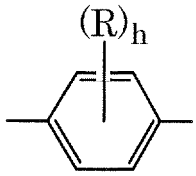

wherein R and h are as defined above, and more preferred is an epoxy compound (1) in which $Ar^1$ and $Ar^3$ independently denote 1,4-phenylene group, 3-methyl-1,4-phenylene group or 3-isopropyl-1,4-phenylene group.

Preferred is an epoxy compound (1) in which $Ar^2$ denotes a group represented by the following formula:

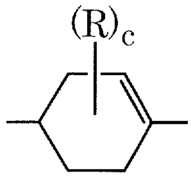

wherein R and c are as defined above, and more preferred is an epoxy compound (1) in which $Ar^2$ denotes 1-cyclohexene-1,4-diyl group.

Among these compounds, more preferred is an epoxy compound (1) in which $Ar^1$ and $Ar^3$ independently denote a group represented by the following formula:

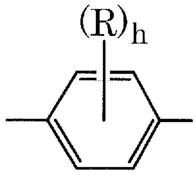

wherein R and h are as defined above, and $Ar^2$ denotes a group represented by the following formula:

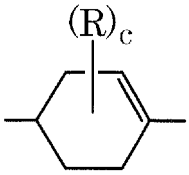

wherein R and c are as defined above, and particularly preferred is an epoxy compound (1) in which $Ar^1$ and $Ar^3$ independently denote 1,4-phenylene group, 3-methyl-1,4-phenylene group or 3-isopropyl-1,4-phenylene group, and $Ar^2$ denotes 1-cyclohexene-1,4-diyl group.

In the above-described formula (1), $R^1$, $R^2$ and $R^3$ are the same or different and each denotes hydrogen atom or an alkyl group having 1 to 8 carbon atoms. Examples of the alkyl group having 1 to 8 carbon atoms include the same groups as those described above.

$Q^1$ denotes a single bond or a straight-chain alkylene group having 1 to 9 carbon atoms. Examples of the straight-chain alkylene group having 1 to 9 carbon atoms include groups formed by bonding 1 to 9 methylene groups linearly, such as methylene, ethylene, trimethylene, tetramethylene, hexamethylene and nonamethylene group. At least one methylene group composing such a straight-chain alkylene group having 1 to 9 carbon atoms is optionally substituted with an alkyl group having 1 to 8 carbon atoms, and -O- or -N($R^4$)- is optionally inserted between the methylene groups.

Examples of the alkyl group having 1 to 8 carbon atoms include the same groups as those described above.

$R^4$ denotes hydrogen atom or an alkyl group having 1 to 8 carbon atoms, and examples of the alkyl group having 1 to 8 carbon atoms include the same groups as those described above.

Examples of the alkylene group in which at least one methylene group is substituted with an alkyl group having 1 to 8 carbon atoms or in which -O- or -N($R^4$)- is inserted between the methylene groups include 2-methyltrimethylene, 1,2-dimethylethylene, 3-oxatetramethylene and 3-oxapentamethylene group.

$Q^1$ is preferably a single bond.

$Q^2$ denotes a straight-chain alkylene group having 1 to 8 carbon atoms and at least one methylene group composing the straight-chain alkylene group is optionally substituted with an alkyl group having 1 to 8 carbon atoms.

Examples of the alkyl group having 1 to 8 carbon atoms and the straight-chain alkylene group having 1 to 8 carbon atoms include the same group as those described above.

Examples of the epoxy compound (1) include

1,4-bis{4-(oxiranylmethoxy)phenyl}cyclohexane,
1-{2-methyl-4-(oxiranylmethoxy)phenyl}-4-{4-(oxiranylmethoxy) phenyl}cyclohexane,
1-{3-methyl-4-(oxiranylmethoxy)phenyl}-4-{4-(oxiranylmethoxy) phenyl}cyclohexane,
1-{3-ethyl-4-(oxiranylmethoxy)phenyl}-4-{4-(oxiranylmethoxy)p henyl}cyclohexane,
1-{3-n-propyl-4-(oxiranylmethoxy)phenyl}-4-{4-(oxiranylmethox y)phenyl}cyclohexane,
1-{3-isopropyl-4-(oxiranylmethoxy)phenyl}-4-{4-(oxiranylmetho xy)phenyl}cyclohexane,
1-{3-n-butyl-4-(oxiranylmethoxy)phenyl}-4-{4-(oxiranylmethoxy )phenyl}cyclohexane,
1-{3-sec-butyl-4-(oxiranylmethoxy)phenyl}-4-{4-(oxiranylmetho xy)phenyl}cyclohexane,
1-{3-tert-butyl-4-(oxiranylmethoxy)phenyl}-4-{4-(oxiranylmeth oxy)phenyl}cyclohexane,
1-{3-n-pentyl-4-(oxiranylmethoxy)phenyl}-4-{4-(oxiranylmethox y)phenyl}cyclohexane,
1-{3-tert-pentyl-4-(oxiranylmethoxy)phenyl}-4-{4-(oxiranylmet hoxy)phenyl}cyclohexane,
1-{3-n-hexyl-4-(oxiranylmethoxy)phenyl}-4-{4-(oxiranylmethoxy )phenyl}cyclohexane,
1-{3-(1-methylpentyl)-4-(oxiranylmethoxy)phenyl}-4-{4-(oxiran ylmethoxy)phenyl}cyclohexane,
1-{3-(1-ethylbutyl)-4-(oxiranylmethoxy)phenyl}-4-{4-(oxiranyl methoxy)phenyl}cyclohexane,
1-{3-cyclohexyl-4-(oxiranylmethoxy)phenyl}-4-{4-(oxiranylmeth oxy)phenyl}cyclohexane,
1-{3-n-heptyl-(oxiranylmethoxy)phenyl}-4-{4-(oxiranylmethoxy) phenyl}cyclohexane,

1-{3-n-octyl-(oxiranylmethoxy)phenyl}-4-{4-(oxiranylmethoxy)p henyl}cyclohexane,
1-{3-(2-ethylhexyl)-(oxiranylmethoxy)phenyl}-4-{4-(oxiranylme thoxy)phenyl}cyclohexane,
1-{3-tert-octyl-(oxiranylmethoxy)phenyl}-4-{4-(oxiranylmethox y)phenyl}cyclohexane,
1-{3-cyclooctyl-(oxiranylmethoxy)phenyl}-4-{4-(oxiranylmethox y)phenyl}cyclohexane,
1-{3-methyl-4-(2-methyl-oxiranylmethoxy)phenyl}-4-{4-(2-methyl -oxiranylmethoxy)phenyl}cyclohexane,
1-{3-methyl-4-(3-methyl-oxiranylmethoxy)phenyl}-4-{4-(3-methyl -oxiranylmethoxy)phenyl}cyclohexane,
1,4-bis{4-(oxiranylmethoxy)phenyl}-1-cyclohexene,
1-{2-methyl-4-(oxiranylmethoxy)phenyl}-4-{4-(oxiranylmethoxy) phenyl}-1-cyclohexene,
1-{3-methyl-4-(oxiranylmethoxy)phenyl}-4-{4-(oxiranylmethoxy) phenyl}-1-cyclohexene,
1-{3-ethyl-4-(oxiranylmethoxy)phenyl}-4-{4-(oxiranylmethoxy)p henyl}-1-cyclohexene,
1-{3-n-propyl-4-(oxiranylmethoxy)phenyl}-4-{4-(oxiranylmethox y)phenyl}-1-cyclohexene,
1-{3-isopropyl-4-(oxiranylmethoxy)phenyl}-4-{4-(oxiranylmetho xy)phenyl}-1-cyclohexene,
1-{3-n-butyl-4-(oxiranylmethoxy)phenyl}-4-{4-(oxiranylmethoxy )phenyl}-1-cyclohexene,
1-{3-sec-butyl-4-(oxiranylmethoxy)phenyl}-4-{4-(oxiranylmetho xy)phenyl}-1-cyclohexene,
1-{3-tert-butyl-4-(oxiranylmethoxy)phenyl}-4-{4-(oxiranylmeth oxy)phenyl}-1-cyclohexene,
1-{3-n-pentyl-4-(oxiranylmethoxy)phenyl}-4-{4-(oxiranylmethox y)phenyl}-1-cyclohexene,
1-{3-tert-pentyl-4-(oxiranylmethoxy)phenyl}-4-{4-(oxiranylmet hoxy)phenyl}-1-cyclohexene,
1-{3-n-hexyl-4-(oxiranylmethoxy)phenyl}-4-{4-(oxiranylmethoxy )phenyl}-1-cyclohexene,
1-{3-(1-methylpentyl)-4-(oxiranylmethoxy)phenyl}-4-{4-(oxiran ylmethoxy)phenyl}-1-cyclohexene,
1-{3-(1-ethylbutyl)-4-(oxiranylmethoxy)phenyl}-4-{4-(oxiranyl methoxy)phenyl}-1-cyclohexene,
1-{3-cyclohexyl-4-(oxiranylmethoxy)phenyl}-4-{4-(oxiranylmeth oxy)phenyl}-1-cyclohexene,
1-{3-n-heptyl-(oxiranylmethoxy)phenyl}-4-{4-(oxiranylmethoxy) phenyl}-1-cyclohexene,
1-{3-n-octyl-(oxiranylmethoxy)phenyl}-4-{4-(oxiranylmethoxy)p henyl}-1-cyclohexene,
1-{3-(2-ethylhexyl)-(oxiranylmethoxy)phenyl}-4-{4-(oxiranylme thoxy)phenyl}-1-cyclohexene,
1-{3-tert-octyl-(oxiranylmethoxy)phenyl}-4-{4-(oxiranylmethox y)phenyl}-1-cyclohexene,
1-{3-cyclooctyl-(oxiranylmethoxy)phenyl}-4-{4-(oxiranylmethox y)phenyl}-1-cyclohexene,
1-{3-methyl-4-(2-methyl-oxiranylmethoxy)phenyl}-4-{4-(2-methyl -oxiranylmethoxy)phenyl}-1-cyclohexene,
1-{3-methyl-4-(3-methyl-oxiranylmethoxy)phenyl}-4-{4-(3-methyl -oxiranylmethoxy)phenyl}-1-cyclohexene,
1,4-bis{4-(oxiranylmethoxy)phenyl}-2-cyclohexene,
1-{2-methyl-4-(oxiranylmethoxy)phenyl}-4-{4-(oxiranylmethoxy) phenyl}-2-cyclohexene,
1-{3-methyl-4-(oxiranylmethoxy)phenyl}-4-{4-(oxiranylmethoxy) phenyl}-2-cyclohexene,
1-{2-methyl-4-(oxiranylmethoxy)phenyl}-4-{4-(oxiranylmethoxy) phenyl}-2-cyclohexene,
1-{3-ethyl-4-(oxiranylmethoxy)phenyl}-4-{4-(oxiranylmethoxy)p henyl}-2-cyclohexene,
1-{3-n-propyl-4-(oxiranylmethoxy)phenyl}-4-{4-(oxiranylmethox y)phenyl}-2-cyclohexene,
1-{3-isopropyl-4-(oxiranylmethoxy)phenyl}-4-{4-(oxiranylmetho xy)phenyl}-2-cyclohexene,
1-{3-n-butyl-4-(oxiranylmethoxy)phenyl}-4-{4-(oxiranylmethoxy )phenyl}-2-cyclohexene,
1-{3-sec-butyl-4-(oxiranylmethoxy)phenyl}-4-{4-(oxiranylmetho xy)phenyl}-2-cyclohexene,
1-{3-tert-butyl-4-(oxiranylmethoxy)phenyl}-4-{4-(oxiranylmeth oxy)phenyl}-2-cyclohexene,
1-{3-n-pentyl-4-(oxiranylmethoxy)phenyl}-4-{4-(oxiranylmethox y)phenyl}-2-cyclohexene,
1-{3-tert-pentyl-4-(oxiranylmethoxy)phenyl}-4-{4-(oxiranylmet hoxy)phenyl}-2-cyclohexene,
1-{3-n-hexyl-4-(oxiranylmethoxy)phenyl}-4-{4-(oxiranylmethoxy )phenyl}-2-cyclohexene,
1-{3-(1-methylpentyl)-4-(oxiranylmethoxy)phenyl}-4-{4-(oxiran ylmethoxy)phenyl}-2-cyclohexene,
1-{3-(1-ethylbutyl)-4-(oxiranylmethoxy)phenyl}-4-{4-(oxiranyl methoxy)phenyl}-2-cyclohexene,
1-{3-cyclohexyl-4-(oxiranylmethoxy)phenyl}-4-{4-(oxiranylmeth oxy)phenyl}-2-cyclohexene,
1-{3-n-heptyl-(oxiranylmethoxy)phenyl}-4-{4-(oxiranylmethoxy) phenyl}-2-cyclohexene,
1-{3-n-octyl-(oxiranylmethoxy)phenyl}-4-{4-(oxiranylmethoxy)p henyl}-2-cyclohexene,
1-{3-(2-ethylhexyl)-(oxiranylmethoxy)phenyl}-4-{4-(oxiranylme thoxy)phenyl}-2-cyclohexene,
1-{3-tert-octyl-(oxiranylmethoxy)phenyl}-4-{4-(oxiranylmethox y)phenyl}-2-cyclohexene,
1-{3-cyclooctyl-(oxiranylmethoxy)phenyl}-4-{4-(oxiranylmethox y)phenyl}-2-cyclohexene,
1-{3-methyl-4-(2-methyl-oxiranylmethoxy)phenyl}-4-{4-(2-methyl -oxiranylmethoxy)phenyl}-2-cyclohexene,
1-{3-methyl-4-(3-methyl-oxiranylmethoxy)phenyl}-4-{4-(3-methyl -oxiranylmethoxy)phenyl}-2-cyclohexene,
1,4-bis{4-(oxiranylmethoxy)phenyl}-2,5-cyclohexadiene,
1-{2-methyl-4-(oxiranylmethoxy)phenyl}-4-{4-(oxiranylmethoxy) phenyl}-2,5-cyclohexadiene,
1-{3-methyl-4-(oxiranylmethoxy)phenyl}-4-{4-(oxiranylmethoxy) phenyl}-2,5-cyclohexadiene,
1-{3-ethyl-4-(oxiranylmethoxy)phenyl}-4-{4-(oxiranylmethoxy)p henyl}-2,5-cyclohexadiene,
1-{3-n-propyl-4-(oxiranylmethoxy)phenyl}-4-{4-(oxiranylmethox y)phenyl}-2,5-cyclohexadiene,
1-{3-isopropyl-4-(oxiranylmethoxy)phenyl}-4-{4-(oxiranylmetho xy)phenyl}-2,5-cyclohexadiene,
1-{3-n-butyl-4-(oxiranylmethoxy)phenyl}-4-{4-(oxiranylmethoxy )phenyl}-2,5-cyclohexadiene,

1-{3-sec-butyl-4-(oxiranylmethoxy)phenyl}-4-{4-(oxiranylmetho xy)phenyl}-2,5-cyclohexadiene,
1-{3-tert-butyl-4-(oxiranylmethoxy)phenyl}-4-{4-(oxiranylmeth oxy)phenyl}-2,5-cyclohexadiene,
1-{3-n-pentyl-4-(oxiranylmethoxy)phenyl}-4-{4-(oxiranylmethox y)phenyl}-2,5-cyclohexadiene,
1-{3-tert-pentyl-4-(oxiranylmethoxy)phenyl}-4-{4-(oxiranylmet hoxy)phenyl}-2,5-cyclohexadiene,
1-{3-n-hexyl-4-(oxiranylmethoxy)phenyl}-4-{4-(oxiranylmethoxy )phenyl}-2,5-cyclohexadiene,
1-{3-(1-methylpentyl)-4-(oxiranylmethoxy)phenyl}-4-{4-(oxiran ylmethoxy)phenyl}-2,5-cyclohexadiene,
1-{3-(1-ethylbutyl)-4-(oxiranylmethoxy)phenyl}-4-{4-(oxiranyl methoxy)phenyl}-2,5-cyclohexadiene,
1-{3-cyclohexyl-4-(oxiranylmethoxy)phenyl}-4-{4-(oxiranylmeth oxy)phenyl}-2,5-cyclohexadiene,
1-{3-n-heptyl-(oxiranylmethoxy)phenyl}-4-{4-(oxiranylmethoxy) phenyl}-2,5-cyclohexadiene,
1-{3-n-octyl-(oxiranylmethoxy)phenyl}-4-{4-(oxiranylmethoxy)p henyl}-2,5-cyclohexadiene,
1-{3-(2-ethylhexyl)-(oxiranylmethoxy)phenyl}-4-{4-(oxiranylme thoxy)phenyl}-2,5-cyclohexadiene,
1-{3-tert-octyl-(oxiranylmethoxy)phenyl}-4-{4-(oxiranylmethox y)phenyl}-2,5-cyclohexadiene,
1-{3-cyclooctyl-(oxiranylmethoxy)phenyl}-4-{4-(oxiranylmethox y)phenyl}-2,5-cyclohexadiene,
1-{3-methyl-4-(2-methyl-oxiranylmethoxy)phenyl}-4-{4-(2-methyl -oxiranylmethoxy)phenyl}-2,5-cyclohexadiene,
1-{3-methyl-4-(3-methyl-oxiranylmethoxy)phenyl}-4-{4-(3-methyl -oxiranylmethoxy)phenyl}-2,5-cyclohexadiene,
1,4-bis{4-(oxiranylmethoxy)phenyl}-1,5-cyclohexadiene,
1-{2-methyl-4-(oxiranylmethoxy)phenyl}-4-{4-(oxiranylmethoxy) phenyl}-1,5-cyclohexadiene,
1-{3-methyl-4-(oxiranylmethoxy)phenyl}-4-{4-(oxiranylmethoxy) phenyl}-1,5-cyclohexadiene,
1-{3-ethyl-4-(oxiranylmethoxy)phenyl}-4-{4-(oxiranylmethoxy)p henyl}-1,5-cyclohexadiene,
1-{3-n-propyl-4-(oxiranylmethoxy)phenyl}-4-{4-(oxiranylmethox y)phenyl}-1,5-cyclohexadiene,
1-{3-isopropyl-4-(oxiranylmethoxy)phenyl}-4-{4-(oxiranylmetho xy)phenyl}-1,5-cyclohexadiene,
1-{3-n-butyl-4-(oxiranylmethoxy)phenyl}-4-{4-(oxiranylmethoxy )phenyl}-1,5-cyclohexadiene,
1-{3-sec-butyl-4-(oxiranylmethoxy)phenyl}-4-{4-(oxiranylmetho xy)phenyl}-1,5-cyclohexadiene,
1-{3-tert-butyl-4-(oxiranylmethoxy)phenyl}-4-{4-(oxiranylmeth oxy)phenyl}-1,5-cyclohexadiene,
1-{3-n-pentyl-4-(oxiranylmethoxy)phenyl}-4-{4-(oxiranylmethox y)phenyl}-1,5-cyclohexadiene,
1-{3-tert-pentyl-4-(oxiranylmethoxy)phenyl}-4-{4-(oxiranylmet hoxy)phenyl}-1,5-cyclohexadiene,
1-{3-n-hexyl-4-(oxiranylmethoxy)phenyl}-4-{4-(oxiranylmethoxy )phenyl}-1,5-cyclohexadiene,
1-{3-(1-methylpentyl)-4-(oxiranylmethoxy)phenyl}-4-{4-(oxiran ylmethoxy)phenyl}-1,5-cyclohexadiene,
1-{3-(1-ethylbutyl)-4-(oxiranylmethoxy)phenyl}-4-{4-(oxiranyl methoxy)phenyl}-1,5-cyclohexadiene,
1-{3-cyclohexyl-4-(oxiranylmethoxy)phenyl}-4-{4-(oxiranylmeth oxy)phenyl}-1,5-cyclohexadiene,
1-{3-n-heptyl-(oxiranylmethoxy)phenyl}-4-{4-(oxiranylmethoxy) phenyl}-1,5-cyclohexadiene,
1-{3-n-octyl-(oxiranylmethoxy)phenyl}-4-{4-(oxiranylmethoxy)p henyl}-1,5-cyclohexadiene,
1-{3-(2-ethylhexyl)-(oxiranylmethoxy)phenyl}-4-{4-(oxiranylme thoxy)phenyl}-1,5-cyclohexadiene,
1-{3-tert-octyl-(oxiranylmethoxy)phenyl}-4-{4-(oxiranylmethox y)phenyl}-1,5-cyclohexadiene,
1-{3-cyclooctyl-(oxiranylmethoxy)phenyl}-4-{4-(oxiranylmethox y)phenyl}-1,5-cyclohexadiene,
1-{3-methyl-4-(2-methyl-oxiranylmethoxy)phenyl}-4-{4-(2-methyl -oxiranylmethoxy)phenyl}-1,5-cyclohexadiene,
1-{3-methyl-4-(3-methyl-oxiranylmethoxy)phenyl}-4-{4-(3-meth -oxiranylmethoxy)phenyl}-1,5-cyclohexadiene,
1,4-bis{4-(oxiranylmethoxy)phenyl}-1,4-cyclohexadiene,
1-{2-methyl-4-(oxiranylmethoxy)phenyl}-4-{4-(oxiranylmethoxy) phenyl}-1,4-cyclohexadiene,
1-{3-methyl-4-(oxiranylmethoxy)phenyl}-4-{4-(oxiranylmethoxy) phenyl}-1,4-cyclohexadiene,
1-{3-ethyl-4-(oxiranylmethoxy)phenyl}-4-{4-(oxiranylmethoxy)p henyl}-1,4-cyclohexadiene,
1-{3-n-propyl-4-(oxiranylmethoxy)phenyl}-4-{4-(oxiranylmethox y)phenyl}-1,4-cyclohexadiene,
1-{3-isopropyl-4-(oxiranylmethoxy)phenyl}-4-{4-(oxiranylmetho xy)phenyl}-1,4-cyclohexadiene,
1-{3-n-butyl-4-(oxiranylmethoxy)phenyl}-4-{4-(oxiranylmethoxy )phenyl}-1,4-cyclohexadiene,
1-{3-sec-butyl-4-(oxiranylmethoxy)phenyl}-4-{4-(oxiranylmetho xy)phenyl}-1,4-cyclohexadiene,
1-{3-tert-butyl-4-(oxiranylmethoxy)phenyl}-4-{4-(oxiranylmeth oxy)phenyl}-1,4-cyclohexadiene,
1-{3-n-pentyl-4-(oxiranylmethoxy)phenyl}-4-{4-(oxiranylmethox y)phenyl}-1,4-cyclohexadiene,
1-{3-tert-pentyl-4-(oxiranylmethoxy)phenyl}-4-{4-(oxiranylmet hoxy)phenyl}-1,4-cyclohexadiene,
1-{3-n-hexyl-4-(oxiranylmethoxy)phenyl}-4-{4-(oxiranylmethoxy )phenyl}-1,4-cyclohexadiene,
1-{3-(1-methylpentyl)-4-(oxiranylmethoxy)phenyl}-4-{4-(oxiran ylmethoxy)phenyl}-1,4-cyclohexadiene,
1-{3-(1-ethylbutyl)-4-(oxiranylmethoxy)phenyl}-4-{4-(oxiranyl methoxy)phenyl}-1,4-cyclohexadiene,
1-{3-cyclohexyl-4-(oxiranylmethoxy)phenyl}-4-{4-(oxiranylmeth oxy)phenyl}-1,4-cyclohexadiene,
1-{3-n-heptyl-(oxiranylmethoxy)phenyl}-4-{4-(oxiranylmethoxy) phenyl}-1,4-cyclohexadiene,
1-{3-n-octyl-(oxiranylmethoxy)phenyl}-4-{4-(oxiranylmethoxy)p henyl}-1,4-cyclohexadiene,
1-{3-(2-ethylhexyl)-(oxiranylmethoxy)phenyl}-4-{4-(oxiranylme thoxy)phenyl}-1,4-cyclohexadiene,
1-{3-tert-octyl-(oxiranylmethoxy)phenyl}-4-{4-(oxiranylmethox y)phenyl}-1,4-cyclohexadiene,
1-{3-cyclooctyl-(oxiranylmethoxy)phenyl}-4-{4-(oxiranylmethox y)phenyl}-1,4-cyclohexadiene,
1-{3-methyl-4-(2-methyl-oxiranylmethoxy)phenyl}-4-{4-(2-methyl -oxiranylmethoxy)phenyl}-1,4-cyclohexadiene,

1-{3-methyl-4-(3-methyl-oxiranylmethoxy)phenyl}-4-{4-(3-methyl -oxiranylmethoxy)phenyl}-1,4-cyclohexadiene,
1,4-bis{4-(oxiranylmethoxy)phenyl}-1,3-cyclohexadiene,
1-{2-methyl-4-(oxiranylmethoxy)phenyl}-4-{4-(oxiranylmethoxy) phenyl}-1,3-cyclohexadiene,
1-{3-methyl-4-(oxiranylmethoxy)phenyl}-4-{4-(oxiranylmethoxy) phenyl}-1,3-cyclohexadiene,
1-{3-ethyl-4-(oxiranylmethoxy)phenyl}-4-{4-(oxiranylmethoxy)p henyl}-1,3-cyclohexadiene,
1-{3-n-propyl-4-(oxiranylmethoxy)phenyl}-4-{4-(oxiranylmethox y)phenyl}-1,3-cyclohexadiene,
1-{3-isopropyl-4-(oxiranylmethoxy)phenyl}-4-{4-(oxiranylmetho xy)phenyl}-1,3-cyclohexadiene,
1-{3-n-butyl-4-(oxiranylmethoxy)phenyl}-4-{4-(oxiranylmethoxy )phenyl}-1,3-cyclohexadiene,
1-{3-sec-butyl-4-(oxiranylmethoxy)phenyl}-4-{4-(oxiranylmetho xy)phenyl}-1,3-cyclohexadiene,
1-{3-tert-butyl-4-(oxiranylmethoxy)phenyl}-4-{4-(oxiranylmeth oxy)phenyl}-1,3-cyclohexadiene,
1-{3-n-pentyl-4-(oxiranylmethoxy)phenyl}-4-{4-(oxiranylmethox y)phenyl}-1,3-cyclohexadiene,
1-{3-tert-pentyl-4-(oxiranylmethoxy)phenyl}-4-{4-(oxiranylmet hoxy)phenyl}-1,3-cyclohexadiene,
1-{3-n-hexyl-4-(oxiranylmethoxy)phenyl}-4-{4-(oxiranylmethoxy )phenyl}-1,3-cyclohexadiene,
1-{3-(1-methylpentyl)-4-(oxiranylmethoxy)phenyl}-4-{4-(oxiran ylmethoxy)phenyl}-1,3-cyclohexadiene,
1-{3-(1-ethylbutyl)-4-(oxiranylmethoxy)phenyl}-4-{4-(oxiranyl methoxy)phenyl}-1,3-cyclohexadiene,
1-{3-cyclohexyl-4-(oxiranylmethoxy)phenyl}-4-{4-(oxiranylmeth oxy)phenyl}-1,3-cyclohexadiene,
1-{3-n-heptyl-(oxiranylmethoxy)phenyl}-4-{4-(oxiranylmethoxy) phenyl}-1,3-cyclohexadiene,
1-{3-n-octyl-(oxiranylmethoxy)phenyl}-4-{4-(oxiranylmethoxy)p henyl}-1,3-cyclohexadiene,
1-{3-(2-ethylhexyl)-(oxiranylmethoxy)phenyl}-4-{4-(oxiranylme thoxy)phenyl}-1,3-cyclohexadiene,
1-{3-tert-octyl-(oxiranylmethoxy)phenyl}-4-{4-(oxiranylmethox y)phenyl}-1,3-cyclohexadiene,
1-{3-cyclooctyl-(oxiranylmethoxy)phenyl}-4-{4-(oxiranylmethox y)phenyl}-1,3-cyclohexadiene,
1-{3-methyl-4-(2-methyl-oxiranylmethoxy)phenyl}-4-{4-(2-methyl -oxiranylmethoxy)phenyl}-1,3-cyclohexadiene,
1-{3-methyl-4-(3-methyl-oxiranylmethoxy)phenyl}-4-{4-(3-methyl -oxiranylmethoxy)phenyl}-1,3-cyclohexadiene,
1,4-bis{4-(oxiranylmethoxy)phenyl}benzene,
1-{2-methyl-4-(oxiranylmethoxy)phenyl}-4-{4-(oxiranylmethoxy) phenyl}benzene,
1-{3-methyl-4-(oxiranylmethoxy)phenyl}-4-{4-(oxiranylmethoxy) phenyl}benzene,
1-{3-ethyl-4-(oxiranylmethoxy)phenyl}-4-{4-(oxiranylmethoxy)p henyl}benzene,
1-{3-n-propyl-4-(oxiranylmethoxy)phenyl}-4-{4-(oxiranylmethox y)phenyl}benzene,
1-{3-isopropyl-4-(oxiranylmethoxy)phenyl}-4-{4-(oxiranylmetho xy)phenyl}benzene,
1-{3-n-butyl-4-(oxiranylmethoxy)phenyl}-4-{4-(oxiranylmethoxy )phenyl}benzene,
1-{3-sec-butyl-4-(oxiranylmethoxy)phenyl}-4-{4-(oxiranylmetho xy)phenyl}benzene,
1-{3-tert-butyl-4-(oxiranylmethoxy)phenyl}-4-{4-(oxiranylmeth oxy)phenyl}benzene,
1-{3-n-pentyl-4-(oxiranylmethoxy)phenyl}-4-{4-(oxiranylmethox y)phenyl}benzene,
1-{3-tert-pentyl-4-(oxiranylmethoxy)phenyl}-4-{4-(oxiranylmet hoxy)phenyl}benzene,
1-{3-n-hexyl-4-(oxiranylmethoxy)phenyl}-4-{4-(oxiranylmethoxy )phenyl}benzene,
1-{3-(1-methylpentyl)-4-(oxiranylmethoxy)phenyl}-4-{4-(oxiran ylmethoxy)phenyl}benzene,
1-{3-(1-ethylbutyl)-4-(oxiranylmethoxy)phenyl}-4-{4-(oxiranyl methoxy)phenyl}benzene,
1-{3-cyclohexyl-4-(oxiranylmethoxy)phenyl}-4-{4-(oxiranylmeth oxy)phenyl}benzene,
1-{3-n-heptyl-(oxiranylmethoxy)phenyl}-4-{4-(oxiranylmethoxy) phenyl}benzene,
1-{3-n-octyl-(oxiranylmethoxy)phenyl}-4-{4-(oxiranylmethoxy)p henyl}benzene,
1-{3-(2-ethylhexyl)-(oxiranylmethoxy)phenyl}-4-{4-(oxiranylme thoxy)phenyl}benzene,
1-{3-tert-octyl-(oxiranylmethoxy)phenyl}-4-{4-(oxiranylmethox y)phenyl}benzene,
1-{3-cyclooctyl-(oxiranylmethoxy)phenyl}-4-{4-(oxiranylmethox y)phenyl}benzene,
1-{3-methyl-4-(2-methyl-oxiranylmethoxy)phenyl}-4-{4-(2-methyl -oxiranylmethoxy)phenyl}benzene,
1-{3-methyl-4-(3-methyl-oxiranylmethoxy)phenyl}-4-{4-(3-methyl -oxiranylmethoxy)phenyl}benzene,
1,4-bis{4-(oxiranylmethoxyethoxy)phenyl}cyclohexane,
1-{3-methyl-4-(oxiranylmethoxyethoxy)phenyl}-4-{4-(oxiranylme thoxyethoxy)phenyl}cyclohexane,
1,4-bis{4-(3-oxiranylpropoxy)phenyl}cyclohexane,
1-{3-methyl-4-(3-oxiranylpropoxy)phenyl}-4-{4-(3-oxiranylprop oxy)phenyl}cyclohexane,
1,4-bis{4-(4-oxiranylbutoxy)phenyl}cyclohexane,
1-{3-methyl-4-(4-oxiranylbutoxy)phenyl}-4-{4-(4-oxiranylbutox y)phenyl}cyclohexane,
1,4-bis{4-(5-oxiranylpentyloxy)phenyl}cyclohexane,
1-{3-methyl-4-(5-oxiranylpentyloxy)phenyl}-4-{4-(5-oxiranylpe ntyloxy)phenyl}cyclohexane,
1,4-bis{4-(6-oxiranylhexyloxy)phenyl}cyclohexane,
1-{3-methyl-4-(6-oxiranylhexyloxy)phenyl}-4-{4-(6-oxiranylhex yloxy)phenyl}cyclohexane,
1,4-bis{4-(8-oxiranyloctyloxy)phenyl}cyclohexane,
1-{3-methyl-4-(8-oxiranyloctyloxy)phenyl}-4-{4-(8-oxiranyloct yloxy)phenyl}cyclohexane,
1,4-bis{4-(oxiranylmethoxyethoxy)phenyl}cyclohexane,

1-{3-methyl-4-(oxiranylmethoxyethoxy)phenyl}-4-{4-(oxiranylme thoxyethoxy)phenyl}cyclohexane,
1,4-bis{4-(2-methyl-oxiranyl)methoxyethoxyphenyl}cyclohexane,
1-{3-methyl-4-(2-methyl-oxiranyl)methoxyethoxyphenyl}-4-{4-(2-methyl-oxiranyl)methoxyethoxyphenyl}cyclohexane,
1,4-bis{4-(3-methyl-oxiranyl)methoxyethoxyphenyl}cyclohexane,
1-{3-methyl-4-(3-methyl-oxiranyl)methoxyethoxyphenyl}-4-{4-(3-methyl-oxiranyl)methoxyethoxyphenyl}cyclohexane,
1,4-bis{4-(oxiranylethoxy)phenyl}-1-cyclohexene,
1-{3-methyl-4-(oxiranylethoxy)phenyl}-4-{4-(oxiranylethoxy)ph enyl}-1-cyclohexene,
1,4-bis{4-(3-oxiranylpropoxy)phenyl}-1-cyclohexene,
1-{3-methyl-4-(3-oxiranylpropoxy)phenyl}-4-{4-(3-oxiranylprop oxy)phenyl}-1-cyclohexene,
1,4-bis{4-(4-oxiranylbutoxy)phenyl}-1-cyclohexene,
1-{3-methyl-4-(4-oxiranylbutoxy)phenyl}-4-{4-(4-oxiranylbutox y)phenyl}-1-cyclohexene,
1,4-bis{4-(5-oxiranylpentyloxy)phenyl}-1-cyclohexene,
1-{3-methyl-4-(5-oxiranylpentyloxy)phenyl}-4-{4-(5-oxiranylpe ntyloxy)phenyl}-1-cyclohexene,
1,4-bis{4-(6-oxiranylhexyloxy)phenyl}-1-cyclohexene,
1-{3-methyl-4-(6-oxiranylhexyloxy)phenyl}-4-{4-(6-oxiranylhex yloxy)phenyl}-1-cyclohexene,
1,4-bis{4-(8-oxiranyloctyloxy)phenyl}-1-cyclohexene,
1-{3-methyl-4-(8-oxiranyloctyloxy)phenyl}-4-{4-(8-oxiranyloct yloxy)phenyl}-1-cyclohexene,
1,4-bis{4-(oxiranylmethoxyethoxy)phenyl}-1-cyclohexene,
1-{3-methyl-4-(oxiranylmethoxyethoxy)phenyl}-4-{4-(oxiranylme thoxyethoxy)phenyl}-1-cyclohexene,
1,4-bis{4-(2-methyl-oxiranyl)methoxyethoxyphenyl}-1-cyclohexe ne,
1-{3-methyl-4-(2-methyl-oxiranyl)methoxyethoxyphenyl}-4-{4-(2-methyl-oxiranyl)methoxyethoxyphenyl}-1-cyclohexene,
1,4-bis{4-(3-methyl-oxiranyl)methoxyethoxyphenyl}-1-cyclohexe ne,
1-{3-methyl-4-(3-methyl-oxiranyl)methoxyethoxyphenyl}-4-{4-(3-methyl-oxiranyl)methoxyethoxyphenyl}-1-cyclohexene,
1,4-bis{4-(oxiranylmethoxyethoxy)phenyl}benzene,
1-{3-methyl-4-(oxiranylmethoxyethoxy)phenyl}-4-{4-(oxiranylme thoxyethoxy)phenyl}benzene,
1,4-bis{4-(3-oxiranylpropoxy)phenyl}benzene,
1-{3-methyl-4-(3-oxiranylpropoxy)phenyl}-4-{4-(3-oxiranylprop oxy)phenyl}benzene,
1,4-bis{4-(4-oxiranylbutoxy)phenyl}benzene,
1-{3-methyl-4-(4-oxiranylbutoxy)phenyl}-4-{4-(4-oxiranylbutox y)phenyl}benzene,
1,4-bis{4-(5-oxiranylpentyloxy)phenyl}benzene,
1-{3-methyl-4-(5-oxiranylpentyloxy)phenyl}-4-{4-(5-oxiranylpe ntyloxy)phenyl}benzene,
1,4-bis{4-(6-oxiranylhexyloxy)phenyl}benzene,
1-{3-methyl-4-(6-oxiranylhexyloxy)phenyl}-4-{4-(6-oxiranylhex yloxy)phenyl}benzene,
1,4-bis{4-(8-oxiranyloctyloxy)phenyl}benzene,
1-{3-methyl-4-(8-oxiranyloctyloxy)phenyl}-4-{4-(8-oxiranyloct yloxy)phenyl}benzene,
1,4-bis{4-(oxiranylmethoxyethoxy)phenyl}benzene,
1-{3-methyl-4-(oxiranylmethoxyethoxy)phenyl}-4-{4-(oxiranylme thoxyethoxy)phenyl}benzene,
1,4-bis{4-(2-methyl-oxiranyl)methoxyethoxyphenyl}benzene,
1-{3-methyl-4-(2-methyl-oxiranyl)methoxyethoxyphenyl}-4-{4-(2 -methyl-oxiranyl)methoxyethoxyphenyl}benzene,
1,4-bis{4-(3-methyl-oxiranyl)methoxyethoxyphenyl}benzene and
1-{3-methyl-4-(3-methyl-oxiranyl)methoxyethoxyphenyl}-4-{4-(3 -methyl-oxiranyl)methoxyethoxyphenyl}benzene.

As described in EP 1698625 A1, a crude product of an epoxy compound, which contains the epoxy compound (1) as a main component, can be produced, for example, by a method comprising reacting a dihydroxy compound represented by the formula (2):

$$HO\text{-}Q^1\text{-}Ar^1\text{-}Ar^2\text{-}Ar^3\text{-}Q^1\text{-}OH \qquad (2)$$

wherein $Q^1$, $Ar^1$, $Ar^2$ and $Ar^3$ are as defined above (hereinafter abbreviated to the dihydroxy compound (2)) with a compound represented by the formula (3):

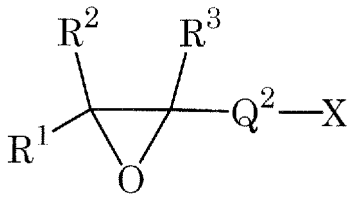

(3)

wherein $R^1$, $R^2$, $R^3$ and $Q^2$ are as defined above, and X denotes a halogen atom (hereinafter abbreviated to the compound (3)) in the presence of a base such as sodium hydroxide, concentrating the obtained reaction mixture to remove the unreacted compound (3), adding a solvent, and optionally washing with water. A crude product of an epoxy compound containing the epoxy compound (1) as a main component can also be produced by a method comprising reacting the dihydroxy compound (2) with the compound (3) in the presence of at least one selected from the group consisting of an amine compound and an ammonium salt, mixing the obtained reaction mixture with an inorganic base to conduct reaction, concentrating the obtained reaction mixture to remove the unreacted compound (3), adding a solvent, and optionally washing with water. When insolubles such as an inorganic salt are precipitated in the obtained crude product, the insolubles are preferably removed by filtration.

When a crude product of an epoxy compound containing the epoxy compound (1) as a main component is produced by reacting the dihydroxy compound (2) with the compound (3) in the presence of at least one selected from the group consisting of an amine compound and an ammonium salt, and mixing the obtained reaction mixture with an inorganic base to further conduct reaction, the inorganic base is usually added to the reaction mixture after dihydroxy compound (2) disappeared as a result of the reaction of the dihydroxy compound (2) and the compound (3). It is preferred to add the inorganic base to the reaction mixture after the dihydroxy compound (2) disappeared and after 50% or more of the compound obtained by reacting one molecule of the obtained dihydroxy compound (2) and one molecule of the compound (3) disappeared. The content of the compound obtained by reacting one molecule of the obtained dihydroxy compound (2) and one molecule of the compound (3) in the reaction mixture can be checked by a conventional analytical means such as liquid chromatography.

Examples of the amine compound include tertiary amines such as trimethylamine, triethylamine, tri-n-propylamine, tri-n-butylamine, N,N-dimethylaniline, N-methylpyrrolidine, N-methylpiperidine and N-methylmorpholine. Examples of the ammonium salt include quaternary ammonium halides such as tetraethylammonium chloride, tetra-n-butylammonium chloride, benzyltriethylammonium chloride, tetraethylammonium bromide and tetra-n-butylammonium bromide. The amount thereof is usually from 0.005 to 20 mol based on 1 mol of the dihydroxy compound (2).

The reaction of the dihydroxy compound (2) and the compound (3) may be carried out in the absence or presence of a solvent. Examples of the solvent include aliphatic hydrocarbon solvents such as n-pentane, n-hexane and n-heptanecyclohexane; aromatic hydrocarbon solvents such as toluene and xylene; alcohol solvents such as methanol, ethanol, n-propanol and isopropanol; glycol solvents such as ethylene glycol; ketone solvents such as acetone and methyl ethyl ketone; nitrile solvents such as acetonitrile; ether solvents such as diethyl ether, tert-butyl methyl ether, 1,4-dioxane and tetrahydrofuran; and amide solvents such as N,N-dimethylformamide. The compound (3) may be used as the solvent.

The reaction temperature is usually from 10 to 100°C.

Examples of the inorganic base include alkali metal hydroxides such as lithium hydroxide, sodium hydroxide and potassium hydroxide; and alkali metal carbonates such as sodium carbonate and potassium carbonate.

The amount of the inorganic base is usually from 0.2 to 20 mol based on 1 mol of the dihydroxy compound (2) used in the reaction of the dihydroxy compound (2) and the compound (3).

The temperature at which the inorganic base is mixed with the reaction mixture, is usually from 0 to 100°C.

As described above, a crude product of an epoxy compound containing the epoxy compound (1) obtained by reacting the dihydroxy compound (2) and the compound (3) as a main component is usually used as the crude product of the epoxy compound containing the epoxy compound (1) as a main component in the present invention. A crude product containing 70% by weight or more of epoxy compound (1) is preferred and a crude product containing 85% by weight or more of epoxy compound (1) is more preferred.

The crude product usually contains the dihydroxy compound (2) as a raw material used, or reaction intermediates. When a dihydroxy compound represented by the formula (4):

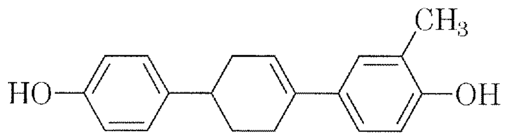 (4)

is used as the dihydroxy compound (2) and epichlorohydrin is used as the compound (3), a crude product containing an epoxy compound represented by the formula (5):

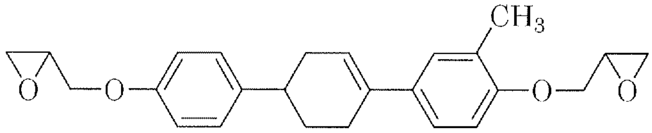 (5)

as a main component is obtained. The crude product contains a dihydroxy compound represented by the formula (4) as a raw material, a compound represented by the following formula (6):

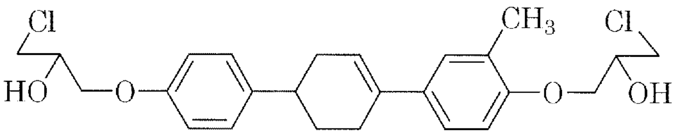 (6)

and a compound represented by the formula (7).

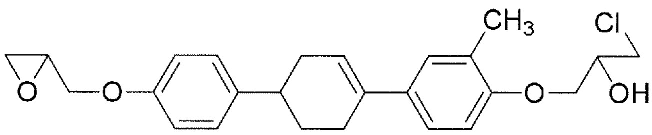 (7)

Examples of the solvent of the solution containing a crude product of the epoxy compound include aliphatic ketone solvents such as acetone, methyl ethyl ketone, diethyl ketone and methyl isobutyl ketone; ether solvents such as diethyl ether, tert-butyl methyl ether, 1,4-dioxane and tetrahydrofuran; aromatic hydrocarbon solvents such as benzene, toluene, ethylbenzene, xylene, cumene, cymene and chlorobenzene; and nitrile solvents such as acetonitrile and propionitrile; or a mixed solvent thereof. At least one selected from the group consisting of aliphatic ketone solvents, ether solvents and aromatic hydrocarbon solvents is preferred. The solution may contain aliphatic hydrocarbon solvents such as n-pentane, n-hexane, 1-hexane, n-heptane, 1-octane, n-octane, n-decane, cyclopentane and cyclohexane.
When the amount of the solvent is too large, the yield of the epoxy compound (1) decreases. In contrast, when the amount is too small, adhesion thereof to the inside of a reaction vessel by scaling is likely to occur. Therefore, a saturation temperature T°C of the epoxy compound (1) in the solution is adjusted within a range from 30 to 60°C according to the kind and amount of the epoxy compound (1) and the kind of the solvent. The concentration of the epoxy compound (1) in the solution is preferably from 10 to 40% by weight. As used herein, the saturation temperature T°C means a temperature when the solution is cooled to precipitate crystals of the epoxy compound (1) and then the entire precipitated crystals are dissolved by heating the solution in which crystals of the obtained epoxy compound (1) are precipitated.
The step (A) is a step of cooling a solution containing a crude product of the epoxy compound, which contains the epoxy compound as a main component, to an internal temperature $T^1$°C to precipitate crystals of the epoxy compound (1), thus obtaining a suspension.
The cooling temperature $T^1$ °C of the solution may be a temperature which is the saturation temperature T°C or lower and at which crystals of the epoxy compound (1) can be precipitated. While it varies depending on the content of the epoxy compound (1) and the kind of the organic solvent, when the concentration of the epoxy compound (1) in

the solution is from 10 to 40% by weight, the cooling temperature $T^1$ °C is preferably a temperature which satisfies the following formula (III):

$$T^1 < T - 5 \qquad (III)$$

and specifically, it is from 10 to 30°C, and preferably from 20 to 30°C.

If necessary, seed crystals may be added in the solution. Preferably, seed crystals are added after the temperature of the solution became saturation temperature T°C or lower. More preferably, seed crystals are added at the stage where the temperature of the solution is within a range from T°C to (T-20) °C and crystals of the epoxy compound (1) are not precipitated.

It is preferred that the suspension cooled to the temperature $T^1$ °C is maintained at the same temperature for about 1 to 3 hours while stirring.

Thus, there can be obtained a suspension in which usually 10 to 80% by weight, and preferably 20 to 60% by weight of the epoxy compound (1) contained in the solution is precipitated in the form of crystals.

Subsequently, the step (B) of heating the suspension obtained in the step (A) to a temperature $T^2$°C, which satisfies the formula (I):

$$T^1 < T^2 < T \qquad (I)$$

wherein T denotes a saturation temperature of an epoxy compound in the solution containing a crude product of the epoxy compound used in the step (A), will be described.

The temperature $T^2$ may be a temperature which is lower than a saturation temperature T°C of the epoxy compound in the solution containing a crude product of the epoxy compound used in the step (A), which is higher than cooling temperature $T^1$ in the step (A), and at which a portion of crystals of the epoxy compound (1) in the suspension can be dissolved. Preferably, the temperature is a temperature at which the suspension wherein about 10 to 50% by weight of the epoxy compound (1) in the used solution is precipitated in the form of crystals is obtained. Specifically, when the concentration of the epoxy compound (1) is from 10 to 40% by weight, the temperature $T^2$ preferably satisfies the following formula (IV).

$$T - 5 < T^2 < T \qquad (IV)$$

Practically, the temperature $T^2$ is preferably from 30 to 60°C, and more preferably from 35 to 45°C.

After heating to $T^2$ °C, the suspension is preferably maintained at the same temperature for about 1 to 4 hours while stirring.

Finally, the step (C) of cooling the suspension heated to $T^2$°C in the step (B) to a temperature $T^3$°C, which satisfies the following formula (II), will be described.

$$T^3 < T^1 \qquad (II)$$

While the temperature $T^3$ may be lower than the cooling temperature $T^1$ in the step (A), it is preferably a temperature at which 75% by weight or more of the epoxy compound (1) in the used solution is precipitated in the form of crystals. Specifically, when the concentration of the epoxy compound (1) is from 10 to 40% by weight, the temperature $T^3$ preferably satieties the following formula (V).

$$-10 < T^3 < T - 5 \qquad (V)$$

From the point of the view of viscosity of the obtained suspension, the temperature $T^3$ is preferably from -10 to 20°C, and more preferably from 0 to 10°C.

Crystals of the epoxy compound (1) can be isolated by filtering the suspension thus obtained. It is preferred that the suspension is cooled to $T^3$°C and maintained at the same temperature for about 1 to 10 hours while stirring, and then filtered.

Purity of the obtained epoxy compound (1) can be analyzed by a conventional analytical means such as high-performance liquid chromatography.

Example

Hereinafter, the present invention is further illustrated in detail by referring to Examples, but the present invention is not limited to Examples. Analysis was carried out by high-performance liquid chromatography and purity of the obtained epoxy compound was calculated by a high-performance liquid chromatography area percentage method.

Reference Example 1

25.0 g of 1-(3-methyl-4-hydroxyphenyl)-4-(4-hydroxyphenyl)-1-cyclohexen e, 244.9 g of epichlorohydrin and 5.8 g of tetra-n-butylammonium bromide were placed in a reaction vessel equipped with a thermometer, a stirrer and a condenser tube. Under a nitrogen atmosphere, the obtained mixture was reacted by heating at 50°C for 10 hours while stirring. The reaction mixture was analyzed by high-performance liquid chromatography. As a result, 1-(3-methyl-4-hydroxyphenyl)-4-(4-hydroxyphenyl)-1-cyclohexen e disappeared and an area percentage value of a compound obtained by reacting one molecule of 1-(3-methyl-4-hydroxyphenyl)-4-(4-hydroxyphenyl)-1-cyclohexen e and one molecule of epichloronydrinone was 0.41%. The obtained reaction mixture was cooled to room temperature and 11.1 g of sodium hydroxide was added thereto, followed by further reaction at room temperature for 2 hours to obtain the reaction mixture containing 1-(3-methyl-4-oxiranylmethoxyphenyl)-4-(4-oxiranylmethoxyphen yl)-1-cyclohexene. Under reduced pressure conditions, the reaction mixture was concentrated at 50°C to remove the unreacted epichlorohydrin. To 78.4 g of the obtained concentrated residue, 245.7 g of methyl isobutyl ketone and 100.8 g of ion-exchange water were added, followed by stirring at 50°C for 30 minutes. After standing, the organic layer and the aqueous layer were separated. The obtained organic layer was washed with 81.1 g of ion-exchange water and concentrated under reduced pressure conditions at 50°C, and then precipitated insolubles were removed by filtration to obtain 250.1 g of a methyl isobutyl ketone solution of 1-(3-methyl-4-oxiranylmethoxyphenyl)-4-(4-oxiranylmethoxyphen yl)-1-cyclohexene (area percentage value of 1-(3-methyl-4-oxiranylmethoxyphenyl)-4-(4-oxiranylmethoxyphen yl)-1-cyclohexene: 96.2%). Saturation temperature was 42°C.

Example 1

250.1 g of a methyl isobutyl ketone solution containing 1-(3-methyl-4-oxiranylmethoxyphenyl)-4-(4-oxiranylmethoxyphen yl)-1-cyclohexene obtained in Reference Example 1 was cooled to 20°C to precipitate crystals, and thus a suspension was obtained. The obtained suspension was heated to 40°C and then maintained at the same temperature for 3 hours. The suspension was cooled to 10°C over 7 hours and then maintained at the same temperature for 2 hours. The obtained suspension was filtered. The filtration time was within one minute and filterability of crystals was extremely excellent. The obtained crystal was washed three times with 20 g of methyl isobutyl ketone adjusted to 10°C and then dried under reduced pressure conditions at 50°C to obtain 27.3 g of 1-(3-methyl-4-oxiranylmethoxyphenyl)-4-(4-oxiranylmethoxyphen yl)-1-cyclohexene as white crystals. Area percentage value: 98.8%, yield: 77.8%.

Example 2

218.8 g of a methyl isobutyl ketone solution (saturation temperature: 42°C) containing 1-(3-methyl-4-oxiranylmethoxyphenyl)-4-(4-oxiranylmethoxyphen yl)-1-cyclohexene obtained in the same manner as in Reference Example 1 was cooled to 20°C to precipitate crystals and thus a suspension was obtained. The obtained suspension was heated to 38°C and then maintained at the same temperature for 3 hours. The suspension was cooled to 10°C over 3.5 hours and then maintained at the same temperature for 2 hours. The obtained suspension was filtered. The filtration time was within one minute and filterability of crystals was extremely excellent. The obtained crystal was washed three times with 20 g of methyl isobutyl ketone adjusted to 10°C and then dried under reduced pressure conditions at 50°C to obtain 24.6 g of 1-(3-methyl-4-oxiranylmethoxyphenyl)-4-(4-oxiranylmethoxyphen yl)-1-cyclohexene as white crystals. Area percentage value: 98.7%, yield: 70.4%.

Example 3

217.7 g of a methyl isobutyl ketone solution (saturation temperature: 42°C) containing 1-(3-methyl-4-oxiranylmethoxyphenyl)-4-(4-oxiranylmethoxyphen yl)-1-cyclohexene obtained in the same manner as in Reference Example 1 and then cooled to 20°C to precipitate crystals, and thus a suspension was obtained. The obtained suspension was heated to 35°C and then maintained at the same temperature for 3 hours. The suspension was cooled to 10°C over 7 hours and then maintained at the same temperature for 2 hours. The obtained suspension was filtered. The filtration time was within one minute and filterability of crystals was extremely excellent. The obtained crystal was washed three times with 20 g of methyl isobutyl ketone adjusted to 10°C and then dried under reduced pressure conditions at 50°C to obtain 23.8 g of 1-(3-methyl-4-oxiranylmethoxyphenyl)-4-(4-oxiranylmethoxyphen yl)-1-cyclohexene as white crystals. Area percentage value: 97.8%, yield: 68.0%.

Reference Example 2

In the same manner as in Reference Example 1, the reaction of 1-(3-methyl-4-hydroxyphenyl)-4-(4-hydroxyphenyl)-1-cyclohexen e and epichlorohydrin was carried out to obtain 94.1 g of a concentrated residue. To the obtained concentrated residue, 200 g of toluene and 100.0 g of ion-exchange water were added, followed by stirring at 50°C for 30 minutes. After standing, the organic layer and the aqueous layer were separated. The obtained organic layer was washed twice with ion-exchange water and then concentrated under reduced pressure conditions at 50°C. The precipitated insolubles were removed by filtration to obtain 218.8 g of a toluene solution containing 1-(3-methyl-4-oxiranylmethoxyphenyl)-4-(4-oxiranylmethoxyphen yl)-1-cyclohexene. Saturation temperature was 40°C.

Example 4

218.8 g of a toluene solution containing 1-(3-methyl-4-oxiranylmethoxyphenyl)-4-(4-oxiranylmethoxyphen yl)-1-cyclohexene obtained in Reference Example 2 was cooled to 20°C to precipitate crystals, thus obtaining a suspension. The obtained suspension was heated to 38°C and then maintained at the same temperature for 3 hours. The suspension was cooled to 10°C over 3.5 hours and then maintained at the same temperature for 2 hours. The obtained suspension was filtered. The filtration time was within one minute and filterability of crystals was extremely excellent. The obtained crystal was washed three times with 20 g of toluene adjusted to 10°C and then dried under reduced pressure conditions at 50°C to obtain 17.9 g of 1-(3-methyl-4-oxiranylmethoxyphenyl)-4-(4-oxiranylmethoxyphen yl)-1-cyclohexene as white crystals. Area percentage value: 98.1%, yield: 51.4%.

Comparative Example 1

260.0 g of a methyl isobutyl ketone solution containing 1-(3-methyl-4-oxiranylmethoxyphenyl)-4-(4-oxiranylmethoxyphen yl)-1-cyclohexene obtained in the same manner as in Reference Example 1 was cooled to 10°C over 3.5 hours and then maintained at the same temperature for 2 hours. The obtained suspension was filtered. As a result, the filtration time was 30 minutes or more and filterability was very poor. The obtained crystals were washed with 20 g of methyl isobutyl ketone adjusted to 10°C and then dried under reduced pressure conditions at 50°C to obtain 27.3 g of 1-(3-methyl-4-oxiranylmethoxyphenyl)-4-(4-oxiranylmethoxyphen yl)-1-cyclohexene as white crystals. Area percentage value: 97.5%, yield: 74.8%.

Comparative Example 2

260.8 g of a methyl isobutyl ketone solution containing 1-(3-methyl-4-oxiranylmethoxyphenyl)-4-(4-oxiranylmethoxyphen yl)-1-cyclohexene obtained in the same manner as in Reference Example 1 was cooled to 10°C over 7 hours and then maintained at the same temperature for 2 hours. The obtained suspension was filtered. As a result, the filtration time was 30 minutes and filterability was very poor. The obtained crystals were washed with 20 g of methyl isobutyl ketone adjusted to 10°C was dried under reduced pressure conditions at 50°C to obtain 27.3 g of 1-(3-methyl-4-oxiranylmethoxyphenyl)-4-(4-oxiranylmethoxyphen yl)-1-cyclohexene as white crystals. Area percentage value: 97.4%, yield: 74.2%.

Industrial Applicability

According to the present invention, an epoxy compound represented by the formula (3), which can form a cured resin that is useful as an insulating material requiring a high thermal conductivity, can be obtained with higher purity and it is industrially advantageous.

## Claims

**1.** A process for crystallizing an epoxy compound, which comprises the steps of:

(A) cooling a solution containing a crude product of an epoxy compound, which contains as a main component the epoxy compound represented by the formula (1):

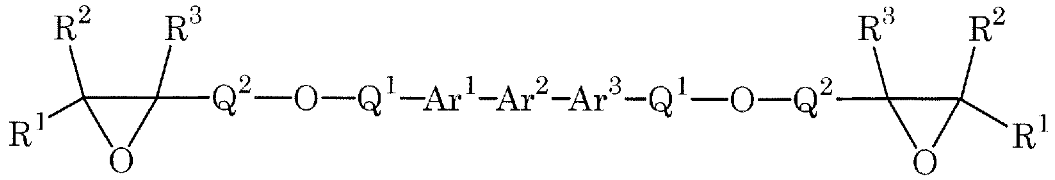

(1)

wherein

$Ar^1$, $Ar^2$ and $Ar^3$ are the same or different and each denotes any one of divalent groups represented by the following formulas:

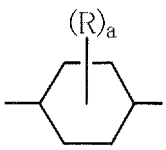 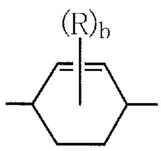 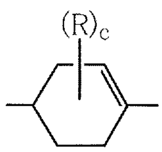 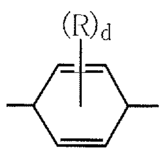

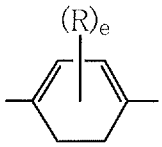 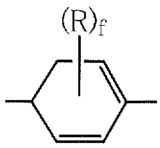 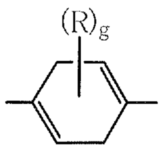 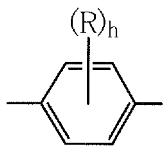

in which R denotes an alkyl group having 1 to 8 carbon atoms, a denotes an integer of 0 to 8, b, e and g denote an integer of 0 to 6, c denotes an integer of 0 to 7, d and h denote an integer of 0 to 4, and f denotes an integer of 0 to 5, and when more than one R exists in said divalent group, all of R may be the same group or different groups;
$R^1$, $R^2$ and $R^3$ are the same or different and each denotes hydrogen atom or an alkyl group having 1 to 8 carbon atoms;
$Q^1$ denotes a single bond or a straight-chain alkylene group having 1 to 9 carbon atoms, in which at least one methylene group composing the straight-chain alkylene group is optionally substituted with an alkyl group having 1 to 8 carbon atoms and -O- or -N ($R^4$)- is optionally inserted between the methylene groups, in which $R^4$ denotes hydrogen atom or an alkyl group having 1 to 8 carbon atoms; and
$Q^2$ denotes a straight-chain alkylene group having 1 to 8 carbon atoms, in which at least one methylene group composing the straight-chain alkylene group is optionally substituted with an alkyl group having 1 to 8 carbon atoms;
to an internal temperature $T^1$°C to precipitate crystals of the epoxy compound represented by the formula (1), thus obtaining a suspension;

(B) heating the suspension obtained in the above-described step (A) to a temperature $T^2$°C which satisfies the formula (I):

$$T^1 < T^2 < T \qquad (I)$$

wherein T denotes a saturation temperature of the epoxy compound in the solution containing a crude product

of the epoxy compound used in the above-described step (A); and
(C) cooling the suspension heated to the temperature $T^2$°C in the above-described step (B) to a temperature $T^3$°C which satisfies the following formula (II).

$$T^3 < T^1 \qquad (II)$$

2. The process for crystallizing an epoxy compound according to claim 1, wherein a solvent of the solution containing a crude product of the epoxy compound is at least one selected from the group consisting of an aliphatic ketone solvent, an ether solvent and an aromatic hydrocarbon solvent.

3. The process for crystallizing an epoxy compound according to claim 1, wherein the temperature T°C is from 30 to 60°C.

4. The process for crystallizing an epoxy compound according to claim 1, wherein the concentration of the epoxy compound in the solution containing a crude product of the epoxy compound is from 10 to 40% by weight and the temperature $T^1$ is a temperature which satisfies the following formula (III).

$$T^1 < T - 5 \qquad (III)$$

5. The process for crystallizing an epoxy compound according to claim 4, wherein the temperature $T^2$ is a temperature which satisfies the following formula (IV).

$$T - 5 < T^2 < T \qquad (IV)$$

6. The process for crystallizing an epoxy compound according to claim 4 or 5, wherein the temperature $T^3$ is a temperature which satisfies the following formula (V):

$$-10 < T^3 < T - 5 \qquad (V)$$

7. The process for crystallizing an epoxy compound according to claim 4, wherein the temperature $T^1$ is from 10 to 30°C.

8. The process for crystallizing an epoxy compound according to claim 5, wherein the temperature $T^2$ is from 30 to 60°C.

9. The process for crystallizing an epoxy compound according to claim 6, wherein the temperature $T^3$ is from -10 to 20°C.

10. The process for crystallizing an epoxy compound according to any one of claims 1 to 3, wherein $Ar^1$ and $Ar^3$ independently denote a group represented by the following formula:

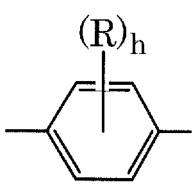

wherein R and h are as defined above.

11. The process for crystallizing an epoxy compound according to claim 10, wherein $Ar^1$ and $Ar^3$ independently denote 1,4-phenylene group, 3-methyl-1,4-phenylene group or 3-isopropyl-1,4-phenylene group.

**12.** The process for crystallizing an epoxy compound according to any one of claims 1 to 3, wherein $Ar^2$ is a group represented by the following formula:

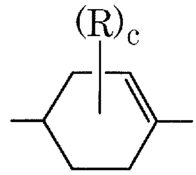

wherein R and c are as defined above.

**13.** The process for crystallizing an epoxy compound according to claim 12, wherein $Ar^2$ is 1-cyclohexene-1,4-diyl group.

**14.** The process for crystallizing an epoxy compound according to any one of claims 1 to 3, wherein $Ar^1$ and $Ar^3$ independently denote a group represented by the following formula:

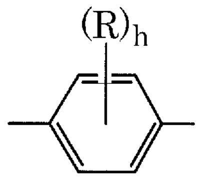

wherein R and h are as defined above, and $Ar^2$ denotes a group represented by the following formula:

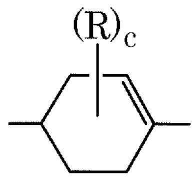

wherein R and c are as defined above.

**15.** The process for crystallizing an epoxy compound according to claim 14, wherein $Ar^1$ and $Ar^2$ independently denote 1,4-phenylene group, 3-methyl-1,4-phenylene group or 3-isopropyl-1,4-phenylene group, and $Ar^2$ denotes 1-cyclohexene-1,4-diyl group.

**INTERNATIONAL SEARCH REPORT**

International application No. PCT/JP2007/055312

A. CLASSIFICATION OF SUBJECT MATTER
*C07D303/30*(2006.01)i, *C07D301/32*(2006.01)i

According to International Patent Classification (IPC) or to both national classification and IPC

B. FIELDS SEARCHED

Minimum documentation searched (classification system followed by classification symbols)
C07D303/30, C07D301/32

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | WO 2005/061473 A1 (Sumitomo Chemical Co., Ltd.), 07 July, 2005 (07.07.05), Claims; examples 1, 2 & EP 1698625 A1 & JP 2005-206814 A | 1-15 |
| Y | JP 62-247802 A (Daicel Chemical Industries, Ltd.), 28 October, 1987 (28.10.87), Claims; page 1, right column, line 12 to page 2, upper left column, line 10; page 2, upper right column, line 19 to lower left column, line 3 (Family: none) | 1-15 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 April, 2007 (03.04.07) | 17 April, 2007 (17.04.07) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2007/055312

C (Continuation). DOCUMENTS CONSIDERED TO BE RELEVANT

| Category* | Citation of document, with indication, where appropriate, of the relevant passages | Relevant to claim No. |
|---|---|---|
| Y | JP 55-034115 A (Kureha Chemical Industry Co., Ltd.), 10 March, 1980 (10.03.80), Claims; page 2, upper left column, lines 8 to 13 (Family: none) | 1-15 |
| A | JP 2004-149472 A (Sumitomo Chemical Co., Ltd.), 27 May, 2004 (27.05.04), (Family: none) | 1-15 |
| A | JP 2005-306858 A (Sumitomo Chemical Co., Ltd.), 04 November, 2005 (04.11.05), & CN 1680387 A & KR 2006044499 A | 1-15 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1698625 A1